# EUROPEAN PATENT SPECIFICATION

(11) **EP 3 994 457 B1**
(45) Date of publication and mention of the grant of the patent: **26.07.2023**
(21) Application number: 20740775.0
(22) Date of filing: 30.06.2020
(51) Int. Cl.: G01N 27/416, G01N 27/30

(54) **PH MEASUREMENT OF AN AQUEOUS SAMPLE WITH A BORON DOPED DIAMOND-BASED PH ELECTRODE COMPRISING A SP2 CARBON REGION**
PH-MESSUNG EINER WÄSSRIGEN PROBE MIT EINER BOR-DOTIERTEN PH-ELEKTRODE AUF DIAMANTBASIS MIT EINEM SP2-KOHLENSTOFFBEREICH
MESURE DU PH D'UN ÉCHANTILLON AQUEUX AVEC UNE ÉLECTRODE DE PH À BASE DE DIAMANT DOPÉ AU BORE COMPRENANT UNE RÉGION DE CARBONE SP2

(30) Priority: 01.07.2019 US 201916459300
(43) Date of publication of application: 11.05.2022
(73) Proprietor: Hach Company, Loveland, CO 80538-8842 (US); UNIVERSITY OF WARWICK, Coventry CV4 8UW (GB)
(72) Inventor: DUNCAN, Zoe, Daventry, Northamptonshire NN11 4AJ (GB); MACPHERSON, Julie, Victoria, Kenilworth CV8 1BE (GB); COBB, Samuel, James, Coventry CV5 8BS (GB)
(74) Representative: Sandri, Sandro
(86) International application number: PCT/US2020/040300
(87) International publication number: WO 2021/003158

(56) References cited:
- WO-A1-2017/223365
- US-A1- 2017 322 172
- ZO? J. AYRES ET AL: "Controlled sp 2 Functionalization of Boron Doped Diamond as a Route for the Fabrication of Robust and Nernstian pH Electrodes", ANALYTICAL CHEMISTRY, vol. 88, no. 1, 5 January 2016 (2016-01-05), pages 974-980, XP055246978, ISSN: 0003-2700, DOI: 10.1021/acs.analchem.5b03732
- TANIA L. READ ET AL: "An sp 2 Patterned Boron Doped Diamond Electrode for the Simultaneous Detection of Dissolved Oxygen and pH", ACS SENSORS, vol. 4, no. 3, 22 February 2019 (2019-02-22), pages 756-763, XP055610398, ISSN: 2379-3694, DOI: 10.1021/acssensors.9b00137

## Description

### FIELD

This application relates to pH measurement of an aqueous sample using electrodes with a carbon region.

### BACKGROUND

Ensuring water quality is critical to the health and well-being of humans, animals, and plants, which are reliant on water for survival. One parameter of water that may be measured is the pH. The measurement of pH of an aqueous sample is critical in a number of industries such as pharmaceuticals, biomedical, water supply, and other manufacturing fields. Measurement of pH may allow for proper treatment of water or ensuring proper water quality for sensitive purposes, and allows for identifying the overall quality of the water. One method to measure pH in an aqueous sample includes the use of electrodes which require constant maintenance and calibration of the pH measurement system.

Document US 2017/0322172 A1 discloses an electrochemical sensor comprising a boron doped diamond electrode formed of boron doped diamond material; an array of non-diamond carbon sites disposed on a sensing surface of the boron doped diamond electrode; electrochemically active surface groups bonded to the non-diamond carbon sites for generating a redox peak associated with a target species which reacts with the electrochemically active surface groups bonded to the non-diamond carbon sites when a solution containing the target species is disposed in contact with the sensing surface in use; an electrical controller configured to scan the boron doped diamond electrode over a potential range to generate said redox peak; and a processor configured to give an electrochemical reading based on one or both of a position and an intensity of said redox peak.

Read et al., An sp2 Patterned Boron Doped Diamond Electrode for the Simultaneous Detection of Dissolved Oxygen and pH, ACS Sensors 2019 4 (3), 756-763 discloses a hybrid sp2-sp3 electrochemical sensor comprising patterned regions of nondiamond-carbon (sp2) in a boron doped diamond (sp3) matrix is described for the simultaneous voltammetric detection of dissolved oxygen (DO) and pH in buffered aqueous solutions.

### BRIEF SUMMARY

In summary, the present invention provides a method for measuring pH in a low conductivity aqueous sample having the features described at claim 1. The dependent claims outline advantageous ways of carrying out the method.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE SEVERAL VIEWS OF THE DRAWINGS

FIG. 1 illustrates an example of computer circuitry.
FIG. 2 illustrates a flow diagram of measuring pH in an aqueous sample.
FIG. 3 illustrates a schematic diagram of a measurement electrode for measuring pH in an aqueous sample.
FIG. 4 illustrates an example of a measurement electrode for measuring pH in an aqueous sample.

### DETAILED DESCRIPTION

The measurement of the pH of water or other aqueous solutions or samples is common and allows for determination of the quality or other characteristics of the aqueous solution. Conventional pH electrodes for measurement of pH may be constructed using fragile, thin glass. This glass breaks easily leading to higher replacement and maintenance costs. The possible breakage of glass pH electrodes may also limit their use in food and beverage applications. Conventional pH electrodes also may have "alkali errors." These errors arise from interfering ions such as sodium and potassium affecting the pH response at high pH values. A commercial need exists for a robust pH measurement electrode that requires less maintenance while maintaining measurement of pH in low conductivity sample.

Another method of measuring pH of an aqueous sample uses a laser machined boron-doped diamond (BDD) material to create a sensor capable of measuring pH. Laser machining of the BDD creates pH sensitive quinone-like structures on the BDD material. These laser machined areas or pits may be in an array pattern. In other words, the laser machined areas may be a series of spots across a surface. The laser machining of these multiple spots may be difficult. For example, an array of laser machines pits across a surface requires using the laser repeatedly across the surface. A single pit or single area of a laser machined area may be an improvement since only one area requires the laser machining. Additionally, the laser may be focused on a surface only once or at least fewer times as compared to laser machining an array of pits. A single pit may have a laser machined cross sectional area similar to the addition of all the laser machined cross sectional areas of an array.

A single pit may give greater accuracy; especially improved accuracy in low conductivity samples. For example, drinking water may have a low conductivity and may require an accurate pH measurement. Low conductivity samples may often be problematic for traditional glass pH electrodes. A single pit BDD-based pH electrode may provide a faster, more accurate pH measurement. A single pit electrode may measure pH in all pH ranges including environmentally relevant range of pH 4 to 11.

A single pit electrode may provide a benefit over time as well. Particulates in a sample may foul an electrode. An array of smaller pits may foul faster as compared to one larger single pit design. Thus, the cost due to maintenance, calibration, replacement, associated personnel hours, or the like may be decreased with a single pit design. An array may have multiple pits that are approximately 45 - 50 µm across. If a sample to be measured has particulate that is of a similar size, then the particulate may foul or clog the array of pits and interfere with measurements. A single pit electrode may have a laser machined area about 370 µm across, minimizing fouling or clogging of a pH-sensitive region. This cross section of the single pit may be altered for different needs and applications. For example, if a particulate size is common in a sample, then the single pit size may be altered to account for particulate size.

Accordingly, the systems and methods as described herein may identify an electrical potential of an aqueous sample using an electrode sensitive to pH in which the conductivity of the sample is low. For example, drinking water which may have a low conductivity. The systems and methods described herein provide a technique for pH measurement using at least one measurement electrode in an aqueous sample. The at least one measurement electrode may have a first carbon region and a single pH sensitive carbon region defined in the first carbon region. The carbon region may be an sp2 carbon region. The pH sensitive carbon region may be laser machined. The laser machining may be a single pit. A single pit may be a single circular area laser machined on the electrode. In an embodiment, the single pit may be of any shape. The carbon region comprises sp2 carbon materials that can include diamond-like materials doped with elements like boron (BDD). The single pH sensitive carbon region is an sp2 carbon region that is included on a boron doped diamond-based pH electrode. Being included may mean that the sp2 carbon region is introduced into, integrated into, contained within, laser micro machined into, or otherwise integrated into the boron doped diamond electrode. In other words, while the sp2 carbon region and the boron doped diamond are integrated into the same electrode, they are chemically different regions of the electrode. The carbon region may have oxidized carbon structures. The oxidized carbon structure may have quinone or quinone-like groups. The method and system also comprises at least one reference electrode and at least one auxiliary electrode. The at least one measurement electrode, at least one reference electrode, and at least one auxiliary electrode may be operatively couple to circuitry to measure, analyze, and store pH measurements of a sample.

The use of BDD serves as a better electrode material than other carbon-based or metallic materials (e.g., silver, gold, mercury, nickel, etc.) because these materials may be more electrocatalytically active, and may generate interfering signals and contributing to the errors in the measurement of pH.

The illustrated example embodiments will be best understood by reference to the figures. The following description is intended only by way of example, and simply illustrates certain example embodiments.

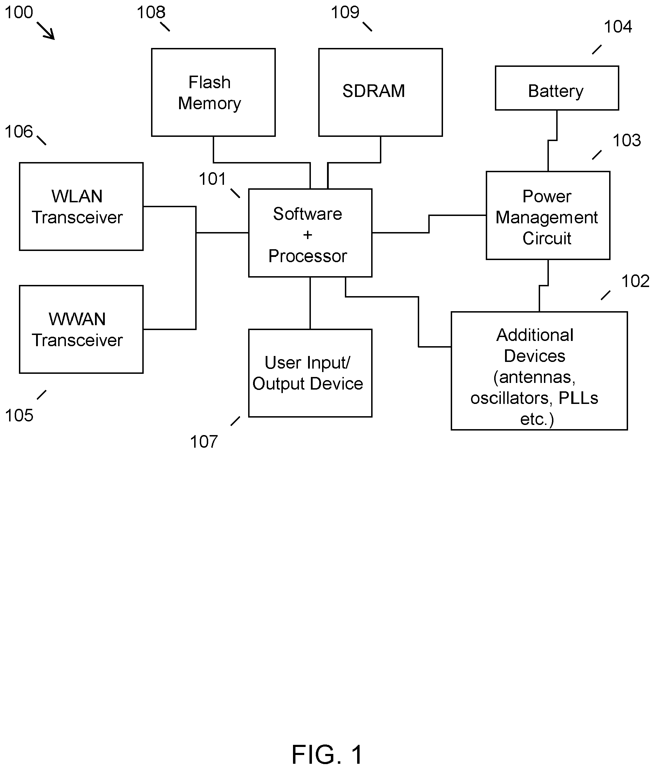

While various other circuits, circuitry or components may be utilized in information handling devices, with regard to an instrument for pH measurement according to any one of the various examples described herein (the circuits, system and devices are not according to the present invention), an example is illustrated in FIG. 1. Device circuitry 100 may include a measurement system on a chip design found, for example, a particular computing platform (e.g., mobile computing, desktop computing, etc.) Software and processor(s) are combined in a single chip 101. Processors comprise internal arithmetic units, registers, cache memory, busses, I/O ports, etc., as is well known in the art. Internal busses and the like depend on different vendors, but essentially all the peripheral devices (102) may attach to a single chip 101. The circuitry 100 combines the processor, memory control, and I/O controller hub all into a single chip 110. Also, systems 100 of this type do not typically use SATA or PCI or LPC. Common interfaces, for example, include SDIO and I2C.

There are power management chip(s) 103, e.g., a battery management unit, BMU, which manage power as supplied, for example, via a rechargeable battery 104, which may be recharged by a connection to a power source (not shown). In at least one design, a single chip, such as 101, is used to supply BIOS like functionality and DRAM memory.

System 100 typically includes one or more of a WWAN transceiver 105 and a WLAN transceiver 106 for connecting to various networks, such as telecommunications networks and wireless Internet devices, e.g., access points. Additionally, devices 102 are commonly included, e.g., a transmit and receive antenna, oscillators, PLLs, etc. System 100 includes input/output devices 107 for data input and display/rendering (e.g., a computing location located away from the single beam system that is easily accessible by a user). System 100 also typically includes various memory devices, for example flash memory 108 and SDRAM 109.

It can be appreciated from the foregoing that electronic components of one or more systems or devices may include, but are not limited to, at least one processing unit, a memory, and a communication bus or communication means that couples various components including the memory to the processing unit(s). A system or device may include or have access to a variety of device readable media. System memory may include device readable storage media in the form of volatile and/or nonvolatile memory such as read only memory (ROM) and/or random access memory (RAM). By way of example, and not limitation, system memory may also include an operating system, application programs, other program modules, and program data. The disclosed system may be used in an embodiment to perform pH measurement of an aqueous sample.

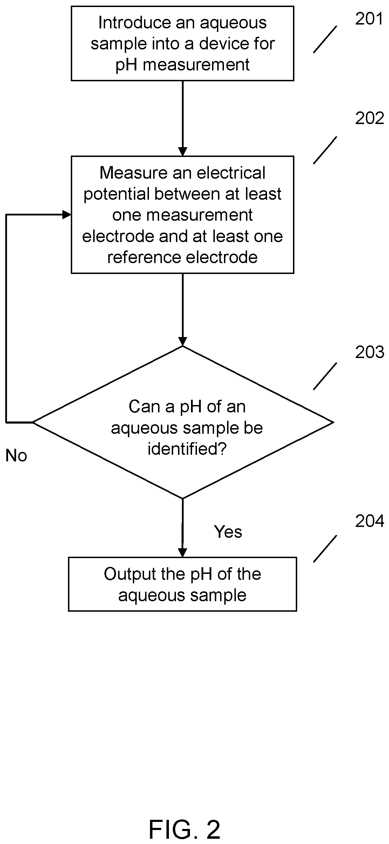

Referring now to FIG. 2, an embodiment measures pH in an aqueous solution using at least one measurement electrode. At least one measurement electrode comprises a pH sensitive carbon region. In other words, a measurement electrode has a region that has been produced by laser machining for example. The pH sensitive region is of a BDD material which comprises sp2 carbon material. The system (not according to the present invention) and method (according to the present invention) also comprises at least one reference electrode and one auxiliary electrode. An applied potential protocol (application of an electrical potential between the at least one reference electrode and the at least one auxiliary electrode) is utilized and a resultant electrical potential is measured between the at least one measurement electrode and at least one reference electrode. The systems (not according to the present invention) and methods (according to the present invention) as described herein provide a technique for accurate measurement of pH in a range of sample types such as low conductivity samples.

At 201, in an embodiment, the aqueous sample is introduced into the measurement device, the aqueous sample is placed or introduced into a test chamber manually by a user or using a mechanical means, for example, gravity flow, a pump, pressure, fluid flow, or the like. For example, a water sample for pH testing is introduced to a measurement or test chamber using a pump. In an embodiment, valves or the like control the influx and efflux of the aqueous solution into or out of the one or more chambers, if present. Once the sample is introduced to the measurement system, the system measures the pH of the sample, using steps as explained in more detail The measurement device may include one or more chambers in which the one or more method steps is performed.

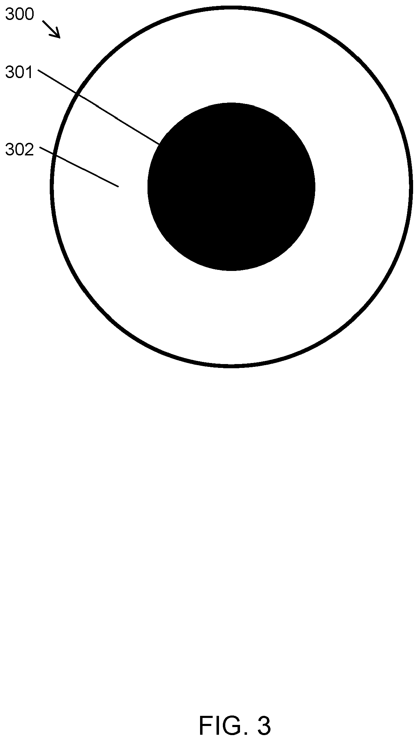

Referring to FIG. 3, the at least one measurement electrode **300** comprises a carbon region **301.** The carbon region is of a BDD material, BDD sp2 material, quinone structures, quinone-like structures, oxidized carbon structures, or the like. The carbon material may be laser machined upon the measurement electrode. The laser machining is to a continuous region. The continuous region may be circular or circular-like in shape such as a circle, oval, or the like. Alternatively, the continuous region may be another geometric shape, for example, square, rectangular, triangular, hexagonal, or the like. In other words, other shapes of carbon regions are disclosed and are laser machined to a particular use or configuration of a measurement electrode.

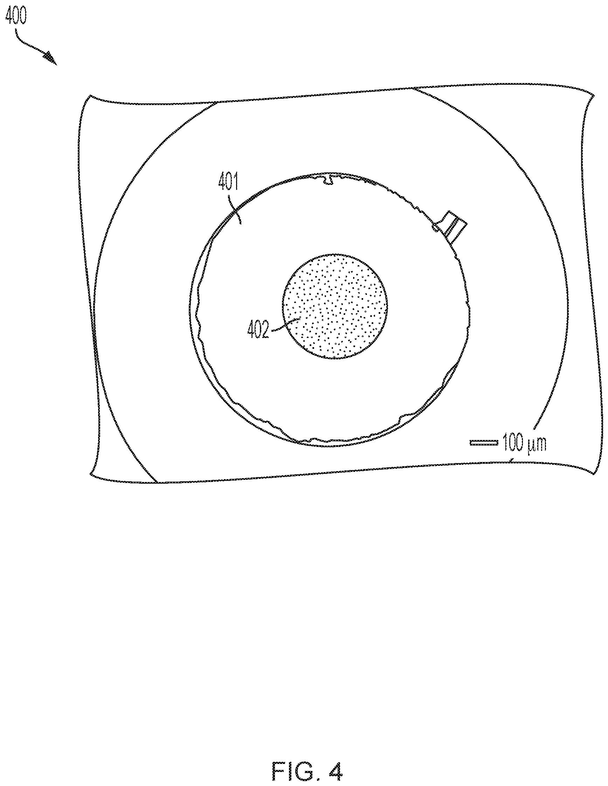

The continuous carbon region may be referred to as a "single pit" measurement electrode. In other words, unlike conventional measurement electrodes that include an array of pits or carbon regions, the electrode of the described system only includes a single pit or carbon region with no other pits or carbon regions on the measurement surface of the electrode. This single pit may be as small as practicable using laser pulse systems or may cover the entirety of the total surface area of the measurement portion of the electrode. In other words, the single pit may be the size of a single laser pulse or as large as the entire face of the electrode. Stated differently, the pH sensitive carbon region **301** may cover an area no larger than the total electrode area exposed to aqueous solution. The total area comprises a pH sensitive carbon region **301** and a non-pH sensitive carbon region **302.** The pH sensitive carbon region **301** may cover an area no smaller than a single laser pulse on the non-pH sensitive carbon region **302** surface area of the measurement electrode. Another view of an example of a measurement electrode **400** for measuring pH in an aqueous sample in an example embodiment is illustrated in FIG. 4. In an embodiment, there may be a BDD region **401** and a laser micromachined BDD region **402.**

The size, depth, or surface area of the laser machined single pit may be constructed for a particular use. In other words, the single pit may be machined in accordance with actual or expected particulate sizes within a sample to be measured. Particulates in a sample may clog or foul a laser machined single pit. The single pit may be laser machined to a size, diameter, depth, or cross section that is larger than particulates present or expected in a sample. In this manner, the laser machine single pit may not foul or clog from particulates present or expected in a pH measurement sample.

The electrodes may be fully or at least partially disposed in the volume of aqueous solution. For example, if the aqueous solution is introduced into a chamber having one or more electrodes, the aqueous solution may at least partially cover the one or more electrodes. As another example, the one or more electrodes may be partially disposed within the chamber with the other portion of the electrode outside the chamber. Thus, when the aqueous solution is introduced into the chamber it only covers the portion of the electrodes that are within the chamber.

At 202, the system measures an electrical potential of the volume of aqueous sample across at least one measurement electrode and at least one reference electrode. The system (not according to the invention) and method (according to the invention) have either, one measurement electrode and one reference electrode, or have a plurality of measurement electrodes and a plurality of reference electrodes.

The electrical potential may be measured across one or more electrodes, for example, a series of electrodes. In an embodiment, the first measurement electrode is used to measure the electrical potential attributable to a pH of an aqueous sample. The at least one measurement electrode contains a carbon region. The carbon region is described above. The carbon region is laser machined. In an embodiment, the carbon region is a BDD, BDD sp2, oxidized carbon structure, quinone, guinone-like structure or the like. The carbon region is a continuous region in a circular-like shape.

If the system cannot identify a pH of the aqueous solution, the system continues to measure electrical responses from the electrodes of the system at 202. Additionally or alternatively, the system may trigger an alarm, shut down, alter flow control of the aqueous sample, or the like. However, if, at 204, a pH of the aqueous sample is determined, the system may output the pH of an aqueous solution. An output may be in the form of a display, storing the data to a memory device, sending the output through a connected or wireless system, printing the output, or the like. The system may be automated, meaning the system may automatically output the identified pH. The system may also have associated alarms, limits, or predetermined thresholds. For example, if a measured pH reaches a threshold, the system may trigger an alarm, adjust the pH of the aqueous solution, alter the flow of the aqueous solution, or the like. Data may be analyzed in real-time, stored for later use, or any combination thereof.

Circuitry controls the measurement (e.g, potential, pH, etc.) to one or more series of electrodes such that different electrical signals are applied and/or measured with respect to the volume of aqueous solution. The circuitry represents an example for at least one measurement electrode with a first carbon region, and a pH sensitive carbon region with at least one reference electrode.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium such as a non-signal storage device, where the instructions are executed by a processor. In the context of this document, a storage device is not a signal and "non-transitory" includes all media except signal media.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and products according to various example embodiments. It will be understood that the actions and functionality may be implemented at least in part by program instructions. These program instructions may be provided to a processor of a device, e.g., a measurement device such as illustrated in FIG. 1, or other programmable data processing device to produce a machine, such that the instructions, which execute via a processor of the device, implement the functions/acts specified.

## Claims

1. A method for measuring pH in a low conductivity aqueous sample with a measurement device comprising at least one measurement electrode (300) having a first carbon region having a single continuous pH sensitive carbon region (301), wherein the single pH sensitive carbon region of the measurement electrode is a single pit sp2 carbon region of a boron doped diamond-based pH electrode, the device further comprising at least one reference electrode, at least one auxiliary electrode and a memory storing instructions executable by a processor to identify a pH of a low conductivity aqueous sample by measuring an electrical potential between the at least one measurement electrode and the at least one reference electrode;
wherein the method comprises:
introducing the low conductivity aqueous sample into the measurement device;
applying an electrical potential to the at least one reference electrode and the at least one auxiliary electrode;
measuring an electrical potential between the at least one measurement electrode and the at least one reference electrode; and
identifying a pH of the low conductivity aqueous sample based upon the electrical potential.

2. The method of claim 1, wherein the low conductivity aqueous sample is constituted by drinking water.

3. The method of any one of the preceding claims, wherein the single pH sensitive carbon region covers a portion of a surface of the at least one measurement electrode.

4. The method of any one of the preceding claims, wherein the pH sensitive carbon region has a defined area.

5. The method of any one of the preceding claims, wherein the single pit electrode is a single laser micromachined pit.

6. The method of any one of the preceding claims, wherein the at least one pH sensitive carbon region of the measurement electrode comprises oxidized carbon structures.

7. The method of claim 6, wherein the oxidized carbon structures further comprise quinone-like groups.

## Patentansprüche

1. Verfahren zum Messen des pH-Wertes in einer wässrigen Probe mit geringer Leitfähigkeit mit einer Messvorrichtung, die mindestens eine Messelektrode (300) mit einem ersten Kohlenstoffbereich mit einem einzelnen kontinuierlichen pH-empfindlichen Kohlenstoffbereich (301) umfasst, wobei der einzelne pH-empfindliche Kohlenstoffbereich der Messelektrode ein einzelner Pit-sp2-Kohlenstoffbereich einer pH-Elektrode auf Basis von bordotiertem Diamant ist, wobei die Vorrichtung ferner mindestens eine Referenzelektrode, mindestens eine Hilfselektrode und einen Speicher umfasst, der Anweisungen speichert, die von einem Prozessor ausgeführt werden können, um einen pH-Wert einer wässrigen Probe mit geringer Leitfähigkeit durch Messen eines elektrischen Potenzials zwischen der mindestens einen Messelektrode und der mindestens einen Referenzelektrode zu ermitteln;
wobei das Verfahren umfasst:
Einbringen der wässrigen Probe mit niedriger Leitfähigkeit in das Messgerät;
Anlegen eines elektrischen Potentials an die mindestens eine Referenzelektrode und die mindestens eine Hilfselektrode;
Messen eines elektrischen Potentials zwischen der mindestens einen Messelektrode und der mindestens einen Referenzelektrode; und
Ermitteln des pH-Wertes der wässrigen Probe mit niedriger Leitfähigkeit auf der Grundlage des elektrischen Potentials.

2. Verfahren nach Anspruch 1, wobei die wässrige Probe mit niedriger Leitfähigkeit aus Trinkwasser besteht.

3. Verfahren nach einem der vorhergehenden Ansprüche, wobei der einzelne pH-empfindliche Kohlenstoffbereich einen Teil einer Oberfläche der mindestens einen Messelektrode bedeckt.

4. Verfahren nach einem der vorhergehenden Ansprüche, wobei der pH-empfindliche Kohlenstoffbereich eine definierte Fläche aufweist.

5. Verfahren nach einem der vorhergehenden Ansprüche, wobei die Einzelpit-Elektrode ein einzelnes lasermikrobearbeitetes Pit ist.

6. Verfahren nach einem der vorhergehenden Ansprüche, wobei der mindestens eine pH-sensitive Kohlenstoffbereich der Messelektrode oxidierte Kohlenstoffstrukturen aufweist.

7. Verfahren nach Anspruch 6, wobei die oxidierten Kohlenstoffstrukturen außerdem chinonartige Gruppen umfassen.

## Revendications

1. Procédé de mesure du pH dans un échantillon aqueux à faible conductivité avec un dispositif de mesure comprenant au moins une électrode de mesure (300) ayant une première région de carbone ayant une région unique et continue de carbone sensible au pH (301), dans lequel la région unique de carbone sensible au pH de l'électrode de mesure est une région de carbone sp2 à puits unique d'une électrode de pH à base de diamant dopé au bore, le dispositif comprenant en outre au moins une électrode de référence, au moins une électrode auxiliaire et une mémoire stockant des instructions exécutables par un processeur pour identifier un pH d'un échantillon aqueux à faible conductivité en mesurant un potentiel électrique entre la au moins une électrode de mesure et la au moins une électrode de référence ;
dans lequel le procédé comprend :
l'introduction de l'échantillon aqueux à faible conductivité dans le dispositif de mesure ;
appliquer un potentiel électrique à la au moins électrode de référence et à la au moins une électrode auxiliaire ;
mesurer un potentiel électrique entre la au moins une électrode de mesure et la au moins une électrode de référence ; et
identifier un pH de l'échantillon aqueux à faible conductivité sur la base du potentiel électrique.

2. Procédé selon la revendication 1, dans lequel l'échantillon aqueux de faible conductivité est constitué par de l'eau potable.

3. Procédé selon l'une quelconque des revendications précédentes, dans lequel la région unique de carbone sensible au pH couvre une partie d'une surface de la au moins une électrode de mesure.

4. Procédé selon l'une quelconque des revendications précédentes, dans lequel la région de carbone sensible au pH a une surface définie.

5. Procédé selon l'une quelconque des revendications précédentes, dans lequel l'électrode à puits unique est un puits unique micro-usiné au laser.

6. Procédé selon l'une quelconque des revendications précédentes, dans lequel la au moins une région de carbone sensible au pH de l'électrode de mesure comprend des structures de carbone oxydé.

7. Procédé selon la revendication 6, dans lequel les structures de carbone oxydé comprennent en outre des groupes de type quinone.
